# EUROPEAN PATENT SPECIFICATION

(11) **EP 2 222 849 B1**
(45) Date of publication and mention of the grant of the patent: **02.11.2011**
(21) Application number: 08863554.5
(22) Date of filing: 18.12.2008
(51) Int. Cl.: C12N 15/10

(54) **METHOD FOR THE IDENTIFICATION OF TARGETS FOR CANCER THERAPY**
VERFAHREN ZUR IDENTIFIZIERUNG VON ZIELMOLEKÜLEN FÜR EINE KREBSTHERAPIE
PROCÉDÉ D'IDENTIFICATION DE CIBLES EN CANCÉROTHÉRAPIE

(30) Priority: 21.12.2007 IT TO20070929
(43) Date of publication of application: 01.09.2010
(73) Proprietor: Medico, Enzo, 10131 Torino (IT)
(72) Inventor: CANTARELLA, Daniela, I-10145 Torino (IT); MARTELLI, Maria, Luisa, I-10146 Torino (IT); MEDICO, Enzo, 10131 Torino (IT)
(74) Representative: Banfi, Paolo
(86) International application number: PCT/EP2008/010856
(87) International publication number: WO 2009/080294

(56) References cited:
- US-A- 5 624 826
- PRITSKER MOSHE ET AL: "Genomewide gain-of-function genetic screen identifies functionally active genes in mouse embryonic stem cells" PROCEEDINGS OF THE NATIONAL ACADEMY OF SCIENCES OF THE UNITED STATES OF AMERICA, vol. 103, no. 18, May 2006 (2006-05), pages 6946-6951, XP002518580 ISSN: 0027-8424
- BERNS K ET AL: "A large-scale RNAi screen in human cells identifies new components of the p53 pathway" NATURE, NATURE PUBLISHING GROUP, LONDON, UK, vol. 428, 25 March 2004 (2004-03-25), pages 431-437, XP003002475 ISSN: 0028-0836
- MARTELLI MARIA LUISA ET AL: "Exploiting orthologue diversity for systematic detection of gain-of-function phenotypes" BMC GENOMICS, BIOMED CENTRAL, LONDON, GB, vol. 9, no. 1, 29 May 2008 (2008-05-29), page 254, XP021032989 ISSN: 1471-2164

## Description

The present invention provides a method for the identification of target genes for cancer therapy.

Identification of a gene whose expression confers neoplastic properties to normal cells, or renders cancer cells resistant to death-promoting stimuli or drugs, directly defines that gene as a potential target for novel therapeutical strategies.

Pritsker Moshe at al (PNAS, vol. 103 no. 18 (2006), p. 6946-6951) disclose a method to identify functional genes in ES cells, through the combination of cDNA library-screens and microarray analyses. This method comprises 1) transfection of cells with cDNA library, 2) selection and propagation at permissive or selective conditions, 3) isolation of library DNA from cells, 4) in vitro biotinylation transcription reaction and 5) microarray analysis of biotinylated cRNA. There is no hint to subjecting a transduced cell population to a selection process, followed by RNA extraction, retrotranscription of said RNA with an oligonucleotide primer specific for vector sequences and microarray analysis.

Functional screenings based on the gain-of-function approach proved extremely valuable in the identification of novel genes involved in key processes related to cancer onset and progression, such as neoplastic transformation, resistance to apoptosis, or escape from senescence (Aruffo et al. Curr Opin Biotechnol. 2:735-741, 1991; Kitamura et al. Exp Hematol. 31: 1007-1014, 2003; Nakayama et al. Curr Opin Biotechnol 3:497-505, 1992).

Screenings of this type are usually performed in mammalian cells by transducing an expression library containing full length cDNAs into a given target cell line. Then a selective stress capable of strongly reducing cell viability and proliferative potential is applied. Only cells expressing exogenous cDNAs conferring resistance to the selection will grow and form resistant colonies (Simonsen & Lodish, Trends Pharmacol Sci. 15:437-441, 1994).

A major drawback, however, is that a huge amount of work is typically required to identify the integrated cDNAs in the resistant colonies, and to verify that they effectively mediate the selective advantage. Moreover, the selection has to be drastic to avoid the emergence of spontaneously resistant colonies, which would dramatically increase the number of false hits.

Thus, there is a need for an alternative method which allows the identification of genes conferring a selective advantage *per se* and potentially synergizing with others, which can be used as potential targets for developing new therapeutic strategies for cancer therapy.

The present invention offers a solution to that problem by providing a screening method as defined in claim 1, an oligonucleotide primer according to claims 10 or 11 and their use according to claim 12.

The method according to the present invention comprises the following steps:
a) transducing a cell population with an expression vector containing a repertoire of DNAs to be analysed as potential target genes;
b) subjecting said population to a selection process and extracting the total RNA;
c) retrotranscribing said total RNA with an oligonucleotide primer specific for vector sequences which are present uniquely in the transcripts deriving from the transduced DNA;
d) detect the transduced DNA by microarray analysis, whereby the presence of transcripts from the transduced DNA, particularly an increased abundance of RNA deriving from transduced DNA after selection, are indicative of a potential target gene for cancer therapy.

This method conveniently utilizes the analysis of gene expression profile to identify genes that confer resistance to specific selection stimuli, with a cause-effect relationship.

As used herein, the expression "repertoire of DNAs" refers to a cDNA expression library, an ORF collection, cDNA or naked DNA.

In a preferred embodiment of the invention, the expression vector is a retroviral vector suitable for the expression of cDNA libraries, such as the Moloney Murine Leukemia Virus (MMLV)-based vectors pFB (Felts et al., Mol Biotechnol. 22:25-32, 2002), pMX and pBabeX (Onishi et al., Exp Hematol. 24:324-329, 1996). Other expression vectors include plasmids like pcDNA3 (Hakvoort et al., Nucleic Acids Res. 24:3478-3480, 1996), and adenoviral vectors, such as pCBII-CMVII (Hillgenberg et al., J Virol. 80:5435-5450, 2006).

In step b), the cell population is selected for resistance to stimuli or conditions that reduce or abolish the cell survival or proliferation capabilities, such as treatment with antineoplastic drugs, with antiproliferative or proapoptotic stimuli, detachment from the substrate or removal of serum or growth factors from the medium.

According to the method of invention, after the selection process has been carried out, the microarray detection of an increased exogenous transcript indicates that the cells transduced with the corresponding DNA have acquired resistance which allowed the cell population to survive the selection stimulus and to proliferate.

As a further advantage, the use of a specific primer which is able to promote exclusively the retrotranscription of the RNA deriving from the transduced DNA, allows to precisely and reliably identify the exogenous DNA expressed in response to the selection process.

Since the method of invention is more sensitive and reliable than known techniques, it does not require extreme conditions of selective stringency or the isolation of resistant clones. Thanks to that, even genes conferring a limited selective advantage can be identified and analysed in detail for potential synergism with others.

In a preferred embodiment, the total RNA extraction is performed, and the abundance of library-derived transcripts is assessed, both before and after applying the selection stimulus. Thus, the transcripts that confer resistance are increased after the selection while those uninvolved in the resistance mechanisms show similar abundance prior to and after the selection.

In a further preferred embodiment, the vector used in the method of invention is a pFB vector, deposited at GenBank under accession number AF132210 (Felts et al., Mol Biotechnol. 22:25-32, 2002).

According to the invention, the primer is designed to promote the specific retrotranscription of mRNAs generated by the transduced DNA and their subsequent *in vitro* transcription by means of T7 RNA polymerase, for the hybridization on DNA microarrays.

In a preferred embodiment, the specific oligonucleotide used in the method of invention contains a sequence complementary to a fragment of the pFB expression vector which is present in all transcripts generated by the vector itself, downstream from the DNA to be tested, and not in transcripts generated by endogenous genes, and has SEQ ID NO:1.

According to the invention, the DNA transduced in the cell population is from an expression library, an ORF collection, cDNA or naked DNA.

In a further preferred embodiment, the species of origin of the exogenous DNA is different from that of the transduced cell population. This difference, in addition to the specificity of the oligonucleotide primer, contributes to the specific detection of the exogenous DNA transcripts. Preferably, both the exogenous DNA and the transduced cell population are of eukaryotic origin.

In a further preferred embodiment, the exogenous DNA (e.g. cDNA) is inserted in the multiple cloning site (MCS) of pFB in 5'-3'orientation. The transcripts deriving from the expression library start at position 616 in the 5' LTR and stop at the end of the 3' LTR, at position 2756. Therefore, besides containing foreign sequences derived from the exogenous DNA, the transcripts display constant motifs derived from the vector. Based on that, a primer specific for pFB was designed, which allows a retrotranscription independent from the sequence of the cDNAs inserted in the MCS. The region located downstream of the MCS was used to design the antisense oligonucleotide sequence. The T7 promoter sequence, which allows the *in vitro* transcription of double-stranded DNA by the T7 RNA polymerase, was added 5' of the pFB-specfic primer sequence; the complete oligonucleotide, named T7-pFB, has sequence SEQ ID NO:1. The main difference with respect to the primer commonly used for retrotranscription (T7-dT(24)), which consists of the T7 sequence followed by a number of 24 dT, is that the latter starts the retrotranscription from the polyA tail which is present in either endogenous or exogenous transcripts, thus resulting less specific and sensitive. Conversely, the use of the T7-pFB primer allowed to increase the amount of RNA in the retrotranscription reaction from 1 to 20 micrograms without increasing the aspecifc hybridization on the microarray.

In a further aspect, the invention provides the use of the oligonucleotide primer described above for the manufacture of a kit for the identification of at least one target gene for cancer therapy.

The following experimental section further illustrates the invention.

### EXPERIMENTAL

### Materials and methods

### Cell culture and reagents

Madin-Darby canine kidney (MDCK) cells and HeLa cells were from ATCC. They were cultured in Dulbecco's modified Eagle's medium (DMEM) (Gibco) supplemented with 10% FBS (Sigma) in a humidified atmosphere of 5% CO2. DEAE-dextran was purchased from Amersham Bioscience.

Polyhema-coated 100 mm Petri dishes were prepared by applying 4 ml of a 12 mg/ml solution of poly-hydroxy-ethyl-methacrylate (polyhema; Sigma) in ethanol, drying under tissue culture hood, repeating the application once and incubating the plates overnight at 37°C.

For the preparation of soft agar plates, SeaPlaque Agarose was used (Cambrex, UK).

### Infections

The mouse testis retroviral expression library, packaged in the VSV envelope was purchased from Stratagene (ViraPort, Cat n. 972300). The library is prepared from a MMLV-like (Moloney Murine Leukemia Virus) replication-defective retroviral vector, named pFB.

Since the number of infective particles present in ViraPort viral surnatants cannot be straightforwardly determined, the viral surnatant obtained from pFB containing a green fluorescent protein coding-sequence (GFP) is supplied with the kit. This allows assessing the infection efficiency of the cells of interest by means of FACS analysis.

As an infection control, 5*10⁴ MDCK and HELA cells were seeded in each well of a 6-wells plate. The following day, from 1:10 to 1:10⁵ dilutions of GFP surnatant were prepared in growth medium supplemented with DEAE-dextran at a final concentration of 10µg/ml. 1 ml of dilution was added to each well and 1 ml of medium without supernatant was added to one well as the control. Plates were then incubated for 3 hrs at 37°C. Afterwards, each well was added with 1ml medium. GFP expression analysis was performed after 48 hours by flow cytometry: cells were trypsinized, diluted in a 1% paraformalhdeide-2% FBS solution and analyzed on a FACS Calibur flow cytometer (Becton Dickinson, San Jose, CA).

The viral titer, expressed as the number of viral particles capable to infect cells in 1ml of surnatant, was calculated as follows: 10³ * % of fluorescent cells (the cells seeded the day before were 5*10⁴, 1*10⁵ at the time of infection) * dilution factor.

The amount of surnatant needed to infect cells is calculated from the titer and from the number of infective particles per cell (MOI= Multiplicity of Infection) required for an experiment.

### Infection titering

To assess the efficiency of the method of invention in detecting library-derived transcripts, HeLa human cells were infected at increasing MOI and the number of exogenous genes detectable by the microarray was determined.

Specifically, 5*10⁴ cells were plated in 4 wells of a 6-well plate. The following day, surnatant-library dilutions corresponding to MOI = 1.25, 2.5 and 5 were prepared in DEAE-dextran supplemented medium at a final concentration of 10µg/ml. 3 wells were added with 1 ml of each dilution and one well was added with the medium without supernatant as a control. The plates were incubated at 37°C for 3 hrs and then 1ml medium was added to each well. After 48 hrs, cells were expanded in 100mm plates and the RNA was extracted using the TRIzol reagent (Invitrogen) according to manufacturer's instructions. The RNA was further purified with Mini kit Rneasy (Qiagen). The quantification and quality analysis of RNA was performed on a Bioanalyzer 2100 (Agilent).

Synthesis of cDNA and biotinylated cRNA was performed using the Illumina TotalPrep RNA Amplification Kit (Ambion Cat. n. IL1791), according to the manufacturer's protocol, with the following variations to optimize microarray analysis: (i) standard cDNA synthesis with the T7-dT(24) primer: 1 µg of total RNA was used, with the doubling of the reaction volume and of all reagents; (ii) library-specific cDNA synthesis: 20 µg of total RNA were used with 4 pm of T7-pFB primer with standard reaction conditions. Quality assessment and quantification of cRNAs were performed on Bioanalyzer 2100.

Hybridization of HeLa-derived cRNAs was carried out for 18 hours on Illumina Mouse 24k arrays (Cat. N. BD-26-201) according to the manufacturer's protocol, using 15 µg of T7-dT(24)-derived cRNA or 1.5 µg of T7-pFB-derived cRNA. Array washing was performed using Illumina High-stringency wash buffer for 30 min at 55°C, and followed by staining and scanning according to standard Illumina protocols. Probe intensity and detection data were obtained using the Illumina BeadStudio software, and further processed with BeadStudio and Microsoft Excel softwares.

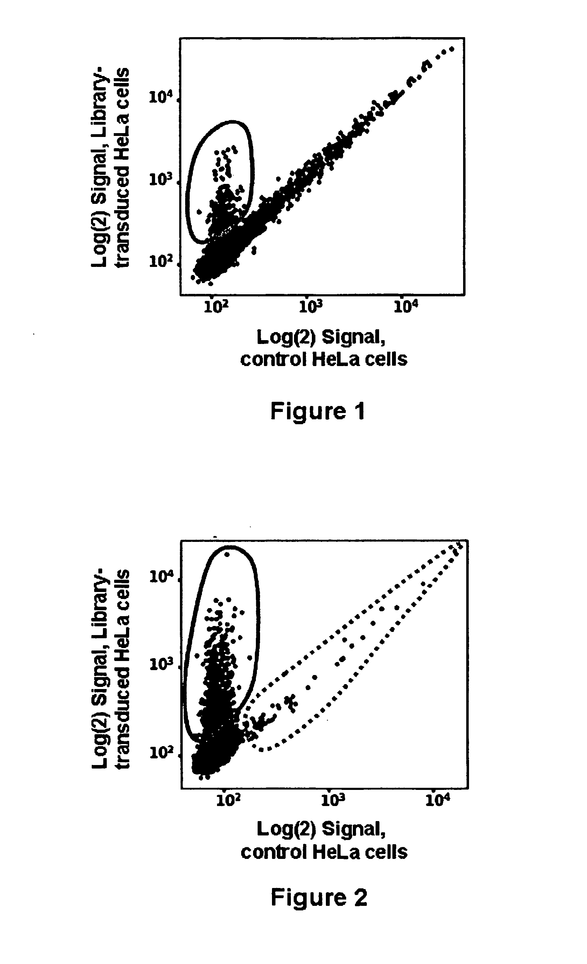

Figure 1 shows the results of the analysis performed on RNAs extracted from HeLa control cells and from cells transduced at MOI~1.25, following the same procedure as for the standard primer. According to this procedure, the number of specifically-detected exogenous transcripts (less than 400) is not sufficient for a functional screening and needs to be improved. In order to increase the number of specifically-detected exogenous transcripts and reduce the hybridization with endogenous transcripts, the procedure described above for the vector-specific T7-pFB primer was followed.

The results obtained with this improved protocol are shown in Figure 2. The number of probes giving significant signal (detection > 0.99) in transduced cells was 1605. Conversely, only 285 probes gave significant signal in untransduced cells, indicating that over 1300 exogenous transcripts were specifically detected in transduced cells.

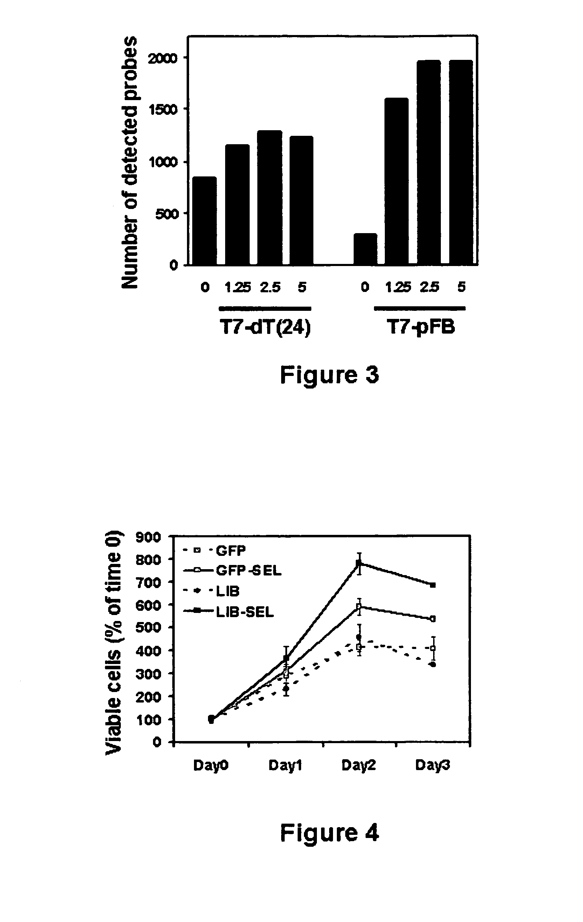

To evaluate whether a higher MOI could increase detection of library-derived genes, the microarray analysis was performed also on the populations transduced at MOI ~2.5 and ~5, using the standard or the T7-pFB primer for cDNA synthesis. The analysis confirmed that use of the T7-pFB primer increased detection of library-derived transcripts also at higher MOIs as shown in Figure 3. Notably, the number of library-derived transcripts reached a plateau at MOI ~2.5 for both primers. Specific detection of almost 1700 murine transcripts at this MOI further confirmed the efficiency of the T7-pFB primer. The number of cDNAs identified by the screening could be increased if the less-abundant cDNA transcripts, which may give undetectable signals in unselected cells, are enriched by subsequent selection steps.

### EXAMPLE

Canine MDCK cells were used for the identification of genes conferring anchorage-loss resistance. Like every non-tumoral epithelial cell, MDCKs grow adhered to the substrate; when these cells are detached and cultured in suspension, for example in plates coated with polyHEMA, proliferation is arrested and the apoptotic process is activated (Frisch and Francis, J Cell Biol. 124:619-626, 1994).

For the functional screening, MDCK cells were transduced with the mouse testis ViraPort retroviral cDNA expression library, or with GFP as a control, at MOI~2.

### a. MDCK infection

1.5*10⁵ MDCK cells were seeded in two 60mm plates for cell culture. The following day, cells from one of the two plates were infected with 1ml non-diluted ViraPort surnatant supplemented with DEAE-dextran at a final concentration of 10µg/ml, corresponding to MOI=2.

The GFP surnatant was added to the control plate. Plates were incubated at 37°C for 3 hrs, then 1.7 ml medium were added to both plates. After 48 hrs the cells were expanded and each plate was divided in two 100mm plates indicated as GFP-A, GFP-B and LIB-A and LIB-B. The four cell populations were then assayed for the expression of library-derived transcripts by means of a microarray. The transcripts were found only in LIB (A and B) populations.

### b. Selection by anchorage-independent growth

1.5*10⁶ MDCK GFP and LIB cells obtained in the previous step were cultured in normal control plates or in 100mm plates previously treated with polyhema for selection and cultured for 3 weeks.

Once a week the cultures were relieved in normal plates for 24 hrs, while every 2-3 days the cells were centrifuged at low speed (400 rpm) and resuspended in fresh medium.

Thus, two selected populations were generated: (1) one GFP-transduced and selected (GFP-SEL) and (2) one library-transduced and selected (LIB-SEL).

The proliferative ability of these populations was then tested either in adhesion conditions or in soft agar.

### c. Soft agar growth assay

In the soft agar growth assay, 5*10⁴ MDCK cells from the four populations described above (GFP, GFP-SEL, LIB and LIB-SEL) were suspended in 1ml growth medium containing 0.5% agar and seeded in duplicate in 6-well plates previuosly coated with 2ml growth medium containing 1% agar. Two weeks later the cells were analysed and photographed in the Leica Multidimensional work-station.

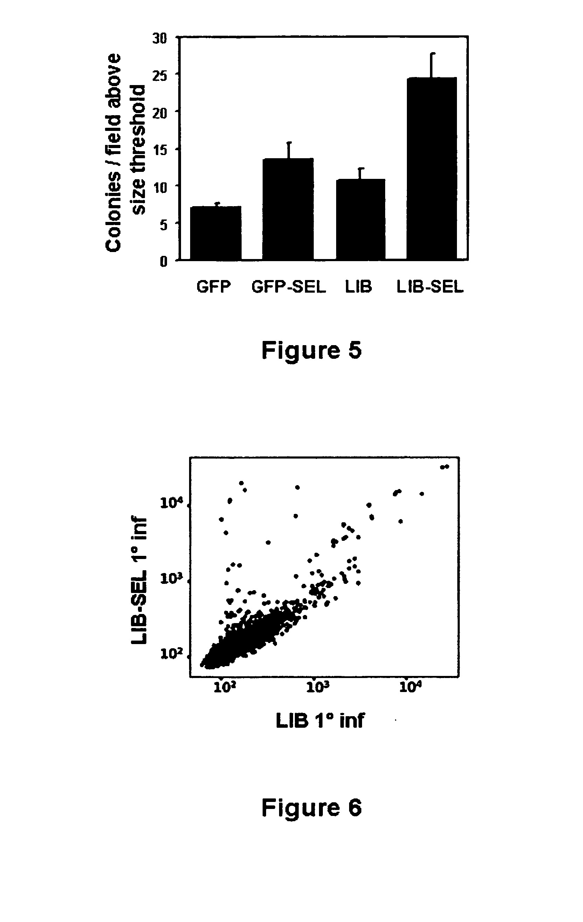

The results are shown in Figures 4 and 5. The LIB-SEL population displayed the highest growth rate in adherence and formed much larger colonies in soft agar. The slight proliferation increase observed also in GFP-SEL population may be due to insertional mutagenesis events that often occur when retroviral vectors are used (Mantovani et al., Nucleic Acids Res. 34: e134, 2006). However such events are not comparable to the effects observed in the LIB-SEL population, which are therefore due to the exogenous cDNAs conferring a selective advantage.

### d. Extraction and processing of the RNA for microarray analysis

In order to identify the transcripts encoded by exogenous cDNAs conferring selective advantage, a microarray analysis was conducted comparing the LIB-SEL population with unselected LIB population. RNA extraction, cDNA and biotinylated cRNA synthesis were performed as described above for the T7-pFB primer.

The cRNAs obtained from MDCK LIB and LIB-SEL cells were hybridized on Illumina Mouse 48k arrays (Cat. N. BD-26-101) using 1.5 µg cRNA synthesized with the specific T7-pFB oligo.

As shown in Fig. 6, a significant number of exogenous transcripts highly expressed and thus enriched was observed in selected cells.

To validate reproducibility of the selective process, a second selection was conducted in parallel on the library-transduced cells described above, whereby the population LIB-SEL-B was generated. Also in this case, the microarray analysis highlighted enriched exogenous transcripts. To verify if the same transcripts were enriched in both selections, we calculated the log2 ratio (L2R) between the signals in selected (LIB-SEL) and unselected (LIB) cells for each transcript in each selection, and compared the results of the two selections.

The analysis revealed that 29 genes, measured by 34 probes on the array, were enriched in both selections (Table 1).

**Table 1**

| Gene | Illumina probe ID | LIB-SEL/LIB selection A enrichment | LIB-SEL-B/LIB selection B enrichment |
|---|---|---|---|
| *Rad9b* | 2320064 | 128.77 | 34.88 |
| *Dhx32* | 2680138 | 103.32 | 49.42 |
| *Mos* | 5290619 | 55.78 | 12.74 |
| *Ndufv1* | 5420369 | 23.51 | 13.24 |
| Hras1 | 102650273 | 15.61 | 60.95 |
| Cct2 | 1690113 | 12.13 | 4.90 |
| Ldh1 | 2190594 | 10.28 | 8.86 |
| Akap4 | 2190731 | 10.02 | 3.59 |
| Hras1 | 1980551 | 6.01 | 20.26 |
| Mcm5 | 2680647 | 5.68 | 20.58 |
| Maea | 105890102 | 5.29 | 22.17 |
| LOC331507 9 | 10476013 | 5.11 | 25.23 |
| LOC271374 | 106400687 | 3.76 | 2.89 |
| Sox6 | 6840717 | 3.64 | 11.39 |
| Ncoa5 | 2060647 | 3.49 | 2.41 |
| Sox5 | 3190128 | 3.20 | 11.25 |
| Temt | 2320020 | 3.07 | 4.14 |
| Fbxo6b | 5690692 | 2.79 | 2.46 |
| Sox5 | 2370576 | 2.78 | 10.70 |
| Eif2b5 | 430315 | 2.10 | 2.77 |
| Hnrpc | 1570133 | 2.01 | 6.22 |
| Eif2b5 | 6900400 | 2.01 | 4.36 |
| 4933424G06Rik | 1240736 | 145.86 | 66.80 |
| 5730438N18Rik | 5340138 | 123.87 | 46.01 |
| 4930528F23Rik | 6620368 | 101.24 | 27.16 |
| 1110003A17Rik | 7000446 | 16.18 | 92.78 |
| 2700067E09Rik | 3440286 | 11.19 | 48.48 |
| 8030459N02Rik | 101450446 | 5.78 | 11.78 |
| 4921511C04Rik | 4070411 | 3.55 | 10.61 |
| 0610007P22Rik | 130193 | 3.42 | 10.02 |
| 2500001K11Rik | 5080577 | 3.20 | 9.36 |
| 0610007P22Rik | 3800433 | 3.16 | 16.36 |
| 0610007P22Rik | 6020750 | 2.39 | 13.03 |
| 2610510J17Rik | 5390500 | 2.26 | 4.23 |

In addition to poorly characterized genes, the screening highlighted proto-oncogenes like *Hras1, Mos* and *Rad-9b,* known to be capable of transforming cells, conferring anchorage independence and/or promoting cell proliferation (Schubbert et al., Nat Rev Cancer 7:295-308, 2007; Singh and Arlinghaus, Mol Carcinog 3:182-189, 1992; Dufault et al., Genomics 6:644-651, 2003).

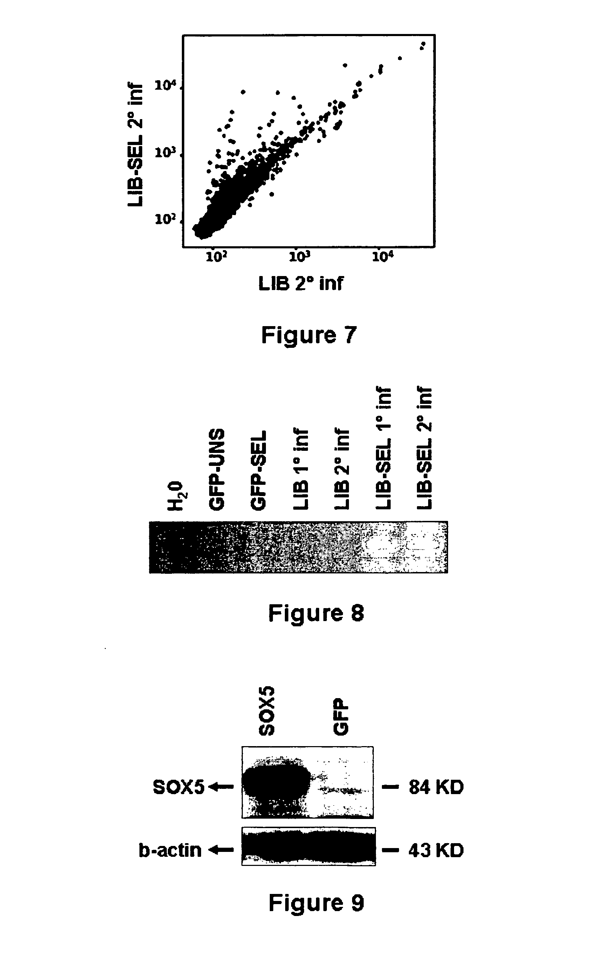

To further validate the screening hits, we performed a second, independent transduction of MDCK cells with the mouse testis library, using a MOI~1 to reduce the possibility of insertional mutagenesis. Cells were selected as described above, and the microarray analysis identified several enriched transcripts, as shown in Figure 7. The transcripts which are reproducibly enriched in both independent screenings are listed in Table 2.

**Table 2**

| Gene | Illumina probe ID | Infection 1 enrichment | Infection 2 enrichment |
|---|---|---|---|
| Sox5 | 3190128 | 3.20 | 42.50 |
| Sox5 | 2370576 | 2.78 | 23.36 |
| Sox6 | 6840717 | 3.64 | 27.78 |
| Hras1 | 102650273 | 15.61 | 4.25 |
| Akap4 | 2190731 | 10.02 | 2.78 |
| 4921511 C04Rik | 4070411 | 3.55 | 2.14 |
| Fbxo6b | 5690692 | 2.79 | 2.58 |
| Hnrpc | 1570133 | 2.01 | 2.80 |

Notably, *Hras1* enrichment was confirmed, albeit less evident, and the *Sox5* and *Sox6* transcription factors displayed the highest concordant enrichment.

### e. Validation of the ability of Sox5 to promote anchorage-independent growth.

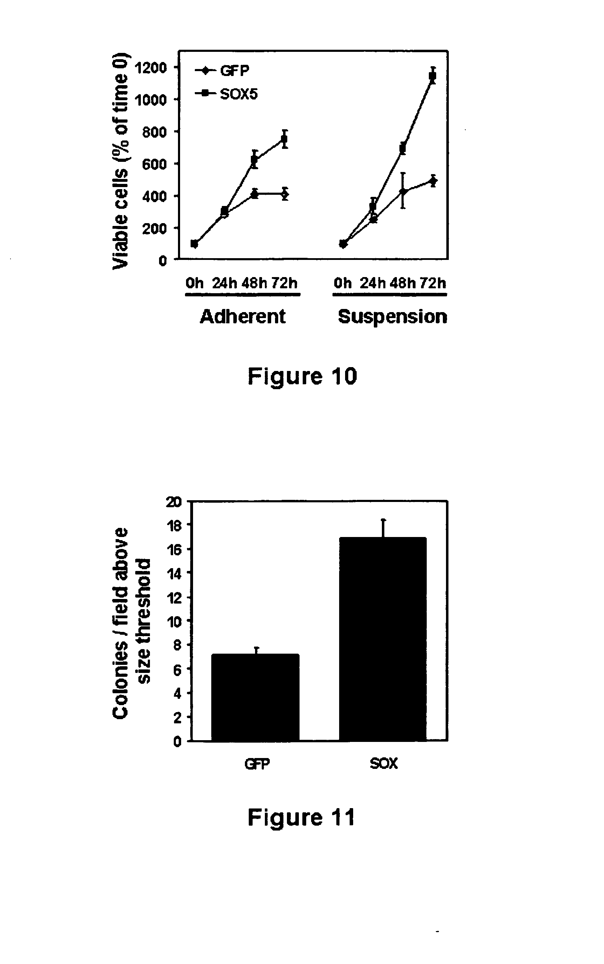

As a further validation of the method of the invention, the transcriptional factor Sox5, when exogenously expressed in MDCK cells, promoted growth in the absence of anchorage. The PCR analysis confirmed that the murine transcript of Sox5 is enriched in library-transduced MDCK cells which have been selected on polyhema, as shown in Figure 8. The Sox5 cDNA was cloned in the retroviral vector pFB (the same used with the library) and transduced in MDCK cells, using pFB-GFP as the control. The expression of the exogenous protein was confirmed by Western blot (Figure 9) and thereafter the Sox5- or GFP-transduced populations were assayed for proliferation on plastics, polyhema or soft agar as shown in Figures 10 and 11. In all the three assays, Sox5-transduced cells showed a higher growth rate. Interestingly, the proliferative advantage was more evident in the absence of anchorage (1.8, 2.3 and 2.4 fold increase for growth on plastics, polyhema and soft agar, respectively). These results confirmed that the use of the methodology proposed here for selective functional screenings allows identifying genes with biological properties determined by the type of selection applied.

## Claims

1. A method *in vitro* for the identification of one or more target genes for cancer therapy, which comprises the following steps:
a) transducing a cell population with an expression vector containing a repertoire of DNAs to be analysed as potential target genes;
b) subjecting said population to a selection process and extracting the total RNA;
c) retrotranscribing said total RNA with an oligonucleotide primer specific for vector sequences which are present uniquely in the transcripts deriving from the transduced DNAs;
d) detecting the transduced DNAs by microarray analysis, whereby the presence of transcripts from the transduced DNA after selection is indicative of a potential target gene for cancer therapy.

2. A method according to claim 1, wherein the total RNA extraction is carried out prior to and after the selection process, whereby an increased abundance of transcripts from the transduced DNA after selection is indicative of potential target genes for cancer therapy.

3. A method according to claim 1, wherein said vector is pFB.

4. A method according to claim 1, wherein said specific oligonucleotide primer contains a sequence complementary to a fragment of the expression vector which is present in all transcripts generated by the vector itself, downstream of the DNA to be tested, and not in transcripts generated by endogenous genes.

5. A method according to claim 4, wherein said primer is an oligonucleotide specific for pFB vector, having sequence SEQ ID NO:1.

6. A method according to claim 1, wherein the transduced DNA is selected from expression library DNA, DNA derived from an ORF collection, cDNA and naked DNA.

7. A method according to claim 1, wherein said cell population and said DNA to be analysed derive from different species, respectively.

8. A method according to claim 7, wherein said cell population derives from an eukaryotic species.

9. A method according to claims 1 and 7, wherein said microarray is specific for the same species from which the transduced DNA derives.

10. An oligonucleotide primer containing a sequence complementary to a portion of the pFB expression vector which is present at the 3' terminals of all transcripts thereof, wherein said oligonucleotide primer has the sequence SEQ ID NO:1.

11. The use of an oligonucleotide primer according to claim 10 for the preparation of a kit for the identification of a target gene for cancer therapy.

## Patentansprüche

1. In-vitro-Verfahren für die Identifizierung eines oder mehrerer Target-Gene für eine Krebstherapie, das die folgenden Schritte umfasst:
a) Transduzieren einer Zellpopulation mit einem Expressionsvektor, enthaltend ein Repertoire von DNAs, die als potentielle Targetgene zu analysieren sind;
b) Unterwerfen der Population einem Selektionsprozess und Extrahieren der Gesamt-RNA;
c) Retrotranskribieren der Gesamt-RNA mit einem Oligonukleotid-Primer, der für Vektorsequenzen spezifisch ist, die in den von den transduzierten DNAs abgeleiteten Transkripten einmal vorkommen;
d) Detektieren der transduzierten DNAs durch Mikroarray-Analyse, wodurch das Vorliegen von Transkripten von der transduzierten DNA nach Selektion indikativ für ein potentielles Target-Gen für eine Krebstherapie ist.

2. Verfahren gemäß Anspruch 1, wobei die Gesamt-RNA-Extraktion vor und nach dem Selektionsprozess durchgeführt wird, wobei eine erhöhte Abundanz von Transkripten von der transduzierten DNA nach Selektion indikativ für potentielle Target-Gene für eine Krebstherapie ist.

3. Verfahren gemäß Anspruch 1, wobei der Vektor pFB ist.

4. Verfahren gemäß Anspruch 1, wobei der spezifisch Oligonukleotid-Primer eine Sequenz enthält, die zu einem Fragment des Expressionsvektors komplementär ist, welches in allen durch den Vektor selbst erzeugten Transkripten stromabwärts der zu testenden DNA und nicht in Transkripten, die durch endogene Gene erzeugt werden, vorliegt.

5. Verfahren gemäß Anspruch 4, wobei der Primer ein Oligonukleotid ist, das für den pFB-Vektor spezifisch ist, das die Sequenz SEQ ID NO:1 hat.

6. Verfahren gemäß Anspruch 1, wobei die transduzierte DNA aus Expressionsbibliothek-DNA, DNA, die aus einer ORF-Sammlung stammt, cDNA und nackter DNA ausgewählt wird.

7. Verfahren gemäß Anspruch 1, wobei die Zellpopulation und die zu analysierende DNA von unterschiedlichen Spezies stammen.

8. Verfahren gemäß Anspruch 7, wobei die Zellpopulation von einer eukaryotischen Spezies stammt.

9. Verfahren gemäß den Ansprüchen 1 und 7, wobei der Mikroarray für dieselbe Spezies spezifisch ist, von der die transduzierte DNA stammt.

10. Oligonukleotid-Primer, der eine Sequenz enthält, die zu einem Teil des pFB-Expressionsvektors komplementär ist, die an den 3'-Termini aller Transkripte davon vorliegt, wobei der Oligonukleotid-Primer die Sequenz SEQ ID NO:1 hat.

11. Verwendung eines Oligonukleotid-Primers gemäß Anspruch 10 für die Herstellung eines Kits für die Identifizierung eines Target-Gens für eine Krebstherapie.

## Revendications

1. Procédé *in vitro* pour l'identification d'un ou plusieurs gènes cibles pour une thérapie de cancer, qui comprend les étapes suivantes :
a) la transduction d'une population cellulaire avec un vecteur d'expression contenant un répertoire d'ADN à analyser en tant que gènes cibles potentiels ;
b) la soumission de ladite population à un processus de sélection et l'extraction de l'ARN total ;
c) la rétrotranscription dudit ARN total avec une amorce oligonucléotidique spécifique pour des séquences de vecteur qui sont exclusivement présentes dans les transcrits dérivant des ADN transduits ;
d) la détection des ADN transduits à l'aide d'une analyse sur puce à ADN, où la présence de transcrits provenant de l'ADN transduit après sélection est indicative d'un potentiel gène cible pour une thérapie de cancer.

2. Procédé selon la revendication 1, dans lequel l'extraction d'ARN total est réalisée avant et après le processus de sélection, où une abondance accrue de transcrits provenant de l'ADN transduit après la sélection est indicative de potentiels gènes cibles pour une thérapie de cancer.

3. Procédé selon la revendication 1, dans lequel ledit vecteur est pFB.

4. Procédé selon la revendication 1, dans lequel ladite amorce oligonucléotidique spécifique contient une séquence complémentaire à un fragment du vecteur d'expression qui est présent dans tous les transcrits générés par le vecteur lui-même, en aval de l'ADN à tester, et pas dans des transcrits générés par des gènes endogènes.

5. Procédé selon la revendication 4, dans lequel ladite amorce est un oligonucléotide spécifique pour le vecteur pFB, ayant la séquence SEQ ID NO 1.

6. Procédé selon la revendication 1, dans lequel l'ADN transduit est choisi parmi un ADN d'une bibliothèque d'ADN d'expression, un ADN dérivé d'une collection d'ORF, un ADNc et un ADN nu.

7. Procédé selon la revendication 1, dans lequel ladite population cellulaire et ledit ADN à analyser dérivent, respectivement, d'une espèce différente.

8. Procédé selon la revendication 7, dans lequel ladite population cellulaire dérive d'une espèce eucaryote.

9. Procédé selon les revendications 1 et 7, dans lequel ladite puce à ADN est spécifique de la même espèce dont dérive l'ADN transduit.

10. Amorce oligonucléotidique contenant une séquence complémentaire à une partie du vecteur d'expression pFB qui est présent au niveau des extrémités terminales 3' de tous les transcrits de celui-ci, dans laquelle ladite amorce oligonucléotide possède la séquence SEQ ID NO : 1.

11. Utilisation d'une amorce oligonucléotidique selon la revendication 10, pour à préparation d'un kit pour l'identification d'un gène cible en vue d'une thérapie de cancer.


### SEQUENCE LISTING

<110> MEDICO, ENZO
<120> METHOD FOR THE IDENTIFICATION OF TARGETS FOR CANCER THERAPY
<130> 1193PCT
<160> 1
<170> PatentIn version 3.3
<210> 1
   <211> 60
   <212> DNA
   <213> Unknown
<220>
   <223> oligonucleotide sequence
<400> 1
   ggccagtgaa ttgtaatacg actcactata gggaggcggc gaaccccaga gtcccgctca 60